# EUROPEAN PATENT APPLICATION

(11) **EP 2 546 228 A1**
(43) Date of publication of application: **16.01.2013**
(21) Application number: 12174265.4
(22) Date of filing: 29.06.2012
(51) Int. Cl.: C07C 51/43, C07C 51/44

(54) **Method for removal of organic compounds from waste water streams in a process for production of (meth)acrylic acid**

(30) Priority: 14.07.2011 US 201161507674 P
(71) Applicant: Rohm and Haas Company, Philadelphia, Pennsylvania 19106-2399 (US)
(72) Inventor: Charendoff, Marc, Houston, TX Texas 77063 (US); Mendoza, Joy, Seabrook, TX Texas 77586 (US); Juliette, James J., Houston, TX Texas 77062 (US); Shah, Rajesh, Katy, TX Texas 77450 (US)
(74) Representative: Buckley, Guy Julian

(57) **Abstract**

The present invention provides a method for removal of organic compounds, such as acetic acid, from waste water streams in processes for production of (meth)acrylic acid. In particular, a mixed product gas, comprising (meth)acrylic acid, acetic acid, propylene and acrolein, is subjected to fractional absorption to produce an aqueous product stream comprising (meth)acrylic acid, water and acetic acid, and an absorber off-gas stream comprising propylene and acrolein. The aqueous product stream is distilled to produce a purified (meth)acrylic acid stream and a waste water stream comprising water and acetic acid. The absorber off gas is then contacted with the waste water stream and at least a portion of the acetic acid moves from the waste water stream to the absorber off gas to produce a stripped waste water stream and an enriched absorber off gas.

## Description

### Field of the Invention

The present invention relates to processes for production of (meth)acrylic acid and, more particularly, to a method for removal of organic compounds from waste water streams in such processes.

### Background of the Invention

The production of (meth)acrylic acid monomers is generally accomplished by vapor phase oxidation of alkanes, alkenes, or mixtures thereof to produce a mixed product gas which contains (meth)acrylic acid as well as various other compounds including unreacted raw materials, by-products, impurities, etc. To separate the (meth)acrylic acid from the other compounds, the mixed product gas may be contacted with an aqueous stream in an absorption step to produce an aqueous (meth)acrylic acid product which comprises mostly (meth)acrylic acid, water and small amounts of the other compounds. An absorber off-gas is also produced by the absorption step and comprises at least some of the other compounds, which are mostly organic compounds, such as, without limitation, unreacted alkane, unreacted alkene, aldehydes and other undesirable volatile organic compounds (VOCs).

Originally, conventional absorption was commonly employed to convert the mixed product gas to the more concentrated aqueous (meth)acrylic acid product (see U.S.

Patent No. 6,399,817). More recently, fractional absorption has been utilized because it more efficiently separates the components of the mixed product gas and, therefore, produces more concentrated aqueous (meth)acrylic acid products which can facilitate downstream purification processes (see, for example, U.S. Patent Nos. 6,482,981 and 7,183,428). In fractional absorption, mixed product gas containing (meth)acrylic acid and aqueous absorbing medium are fed to a fractional absorber wherein (meth)acrylic acid and higher boiling point components of the mixed product gas are absorbed into the aqueous liquid phase to produce an aqueous (meth)acrylic acid stream which exits from the bottom of the fractional absorber, while the lightest components, such as acetic acid, are retained in the gas phase and exit from the top as the absorber off-gas.

Regardless of the particular absorption method, the resulting aqueous (meth)acrylic acid product is often subjected to further purification processes, such as distillation, stripping, fractionation, rectification, etc., in one or more separation apparati, for removal of additional amounts of water, unreacted compounds and other impurities, to produce a (meth)acrylic acid product having the desired purity. Many of the purification steps result in production of waste streams comprising mostly water, which is why they are commonly referred to as waste water streams, although they also contain minor amounts of organic compounds derived from the above-described oxidation and absorption processes (see, for example, U.S. Patent Nos. 6,399,817, 6,482,981 and 7,183,428).

Common practice was to simply discharge the waste water streams to streams, rivers, lakes or ponds, but environmental concerns led to regulations requiring pretreatment of process waste water to first remove or render inert environmentally harmful compounds contained therein. Thus, discarding process waste water has added costs to the overall process operation. To reduce such disposal costs as well as the environmental hazards, it became more common to recycle waste water streams back to various steps in the manufacturing process, such as to the oxidation reactor or the absorber (see, U.S. Patent No. 5,248,819 and 6,399,817). Additionally, waste water streams have been treated prior to recycle or discard, to recover and remove organic components which may be used elsewhere or directed to a more economical disposal method. It is also known to recycle the absorber off-gas to the oxidation reactor or back to the absorber itself (see, U.S. Patent Nos. 5,248,819 and 6,677,482).

Efforts continue to identify and develop methods and techniques by which the waste streams of (meth)acrylic acid production processes can be more efficiently used, reused, treated and discarded. The method of the present invention increases the efficiency of such production processes by using the absorber off-gas from a fractional absorber in a (meth)acrylic acid production process as stripping gas to strip organic compounds from waste water streams prior to recycle or discharge.

### Summary of the Invention

The present invention provides a method for removing acetic acid from a waste water stream which is produced during purification of (meth)acrylic acid. The method involves first performing fractional absorption with a mixed gas comprising (meth)acrylic acid, acetic acid, propylene and acrolein to produce an aqueous product stream comprising (meth)acrylic acid, water and acetic acid, and an absorber off-gas stream comprising propylene and acrolein. Next, the aqueous product stream is distilled to produce a purified (meth)acrylic acid stream and a waste water stream comprising water and acetic acid. The method of the present invention further involves contacting the absorber off gas and the waste water stream whereby at least a portion of the acetic acid moves from the waste water stream to the absorber off gas to produce a stripped waste water stream having a decreased acetic acid content and an enriched absorber off gas having an increased acetic acid content. The enriched absorber off gas stream is provided to a thermal oxidizer.

The stripped waste water stream may be discarded or subjected to further processing to remove or render inert one or more components therein. All or a portion of the stripped waste water stream may fed to at least one other process step.

### Brief Description of the Drawings

A more complete understanding of the present invention will be gained from the embodiments discussed hereinafter and with reference to the accompanying drawings, wherein:
Figure 1 is a schematic representation of a process for purification of (meth)acrylic acid to which the method of the present invention has been applied; and
Figure 2 shows a near linear increase in stripping efficiency as the flow increases.

### Detailed Description of the Invention

All percentages stated herein are weight percentages, unless otherwise specified. As used herein, the term "(meth)acrylic acid" means either acrylic acid or methacrylic acid, or both.

As used herein, the term "(meth)acrolein" means either acrolein or methacrolein. The term "stripping," as used herein, refers to the contacting of a stripping gas with a solution containing target substances so as to migrate one or more target substances from the solution to the gas phase.

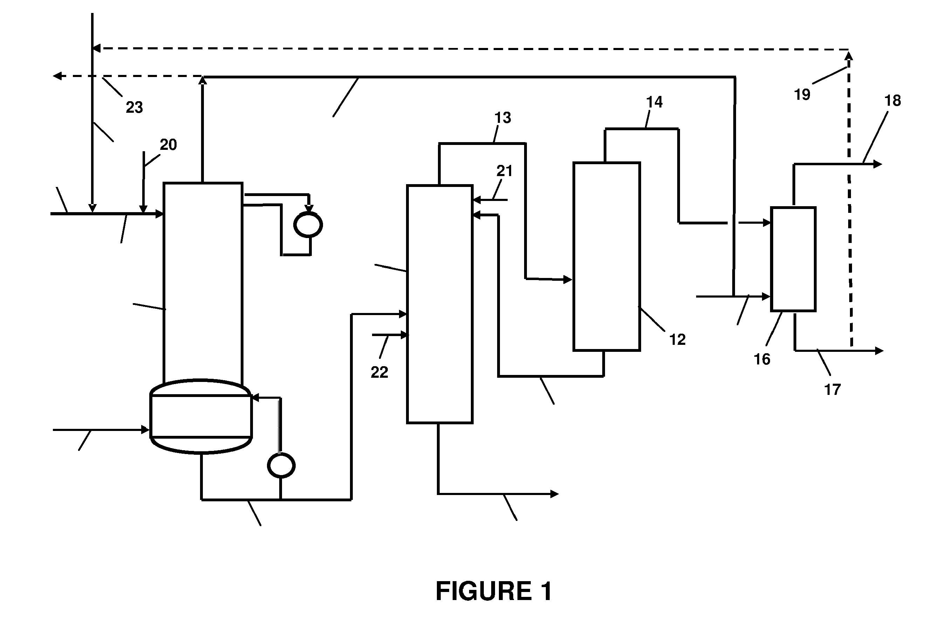

The method of the present invention will be described with reference to Figure 1 which provides a general schematic diagram of a typical (meth)acrylic acid production process wherein the method of the present invention has been implemented.

With reference to Figure 1, in a process for producing (meth)acrylic acid, a mixed product gas **1** is fed to a fractional absorber **2** to separate and recover (meth)acrylic acid from the mixed product gas **1.** While not particularly limited, the mixed product gas **1** may, for example, be obtained by the vapor phase catalytic oxidation of a hydrocarbon material with a molecular oxygen containing gas in the presence of a suitable oxidation catalyst. The vapor phase catalytic oxidation of a hydrocarbon material to (meth)acrolein and/or (meth)acrylic acid as well as reactors, catalysts, and processes for performing the same are generally known in the art and are described, for instance in U.S. Patent Nos. 4,203,906; 4,256,783; 4,365,087; 4,873,368; 5,161,605; 5,177,260; 5,198,578; 5,739,391; and 5,821,390.

Depending on the reactants fed to the reactor, the mixed product gas **1** generally includes inert gas(es), including but not limited to, nitrogen, helium, argon, etc.; acrylic acid; unreacted hydrocarbon reactants, including but not limited to, propylene, acrolein, propane, etc.; steam, and molecular oxygen containing reactants, including but not limited to, air, oxygen, etc.; reaction by-products, including but not limited to, acetic acid, formaldehyde, maleic acid, and other organics; as well as CO₂ CO and H₂O.

Generally, the composition of the mixed product gas **1** includes from 5 to 30 % by weight acrylic acid, from 0.1 to 3.0 % by weight acetic acid, from 0.02 to 0.2 % by weight acrolein, from 30 to 95 % by weight inert gas, and from 1 to 30 % by weight steam, based on the total weight of the mixed product gas **1.**

As shown in Figure 1, an aqueous stream **3** which includes recycled wastewater is also fed to the fractional absorber **2.** The wastewater may be any wastewater suitable for use in absorbing (meth)acrylic acid from a mixed product gas and may be from any source. Consequently, it is not necessary that the wastewater be derived from the same (meth)acrylic acid process stream into which it is recycled. Rather, the wastewater may be derived from one (meth)acrylic acid process stream and recycled into another. Suitable examples of wastewater include, but are not limited to, wastewater derived from dehydration of (meth)acrylic acid, other aqueous distillates, and raffinates. In a like manner, it is not necessary that the wastewater be derived from a (meth)acrylic acid wastewater stream. Accordingly, the wastewater may be derived from other chemical process wastewater streams. Furthermore, the wastewater may be derived from a natural source such as a river, well, spring or the like.

The aqueous stream **3** may include any suitable amount of recycled wastewater **4** up to 100 weight percent recycled wastewater. Typically, the aqueous stream **3** will be a mixture of a wastewater stream **4** from an acrylic acid manufacturing process and an essentially pure water stream **5,** for example, deionized water. In one embodiment, for example, the aqueous stream **3** includes a major amount of wastewater. In another embodiment, the aqueous stream **3** includes from 0.1 % to 100 % by weight of wastewater. Preferably, the aqueous stream **3** contains 100 % by weight wastewater. Regardless of how much recycled wastewater is utilized, the aqueous stream **3** will contain a major amount of water and minor amounts of at least one of acrylic acid, acetic acid, and distillation solvent(s). Generally, the aqueous stream contains less than 3.0 %, preferably less than 2.0 %, more preferably less than 1.5 % by weight acetic acid. In another embodiment, the aqueous stream is substantially free of distillation solvent(s) and/or acrylic acid. With reference still to Figure **1****,** in the fractional absorber **2** the mixed product gas **1** is contacted with the aqueous stream **3** to form an aqueous (meth)acrylic acid stream **9.** The aqueous (meth)acrylic acid stream **9** formed generally includes from 20 to 95 %, preferably 35 to 90 %, and more preferably 50 to 80 % by weight (meth)acrylic acid; from 80 to 5 %, preferably from 65 to 10 %, more preferably from 50 to 20 % by weight water; and up to 8 %, preferably up to 6 %, more preferably up to 5 % by weight acetic acid.

Generally, the mixed product gas **1** is fed to the fractional absorber **2** at a temperature from 165 °C to 400 °C, preferably 200 °C to 350 °C, more preferably 250 °C to 325 °C. The aqueous stream **3** is fed to the fractional absorber **2** at a rate of 0.1 to 1.0 pounds of aqueous stream **3** per one pound of hydrocarbon material (in mixed gas product stream **1)** fed to the reactor depending on the desired concentration of acrylic acid to be recovered from the bottoms of the fractional absorber **2.** The fractional absorber **2** may be any suitable fractional absorber design known in the art in which (meth)acrylic acid and higher boiling point components of the mixed product gas are absorbed into the aqueous liquid phase to produce the aqueous (meth)acrylic acid stream **9,** while the lightest components, such as acetic acid, are retained in the gas phase and exit from the top as the absorber off-gas **8.**

To concentrate the (meth)acrylic acid, the aqueous (meth)acrylic acid stream **9** is fed to one or more distillation columns, such as the product column **10** and the subsequent acetic acid recovery column **12** shown schematically in Figure **1****.** The distillation columns **10, 12** may be any suitable distillation column known in the art. For instance, sieve trays, dual flow tray design, or packed column design may be used.

The aqueous (meth)acrylic acid stream **9** is distilled in the product column **10** to form a (meth)acrylic acid stream **11** and an intermediate waste stream **13** comprising water, acetic acid and other organic compounds. The distillation performed in one or both of columns **10, 12** may be simple, non-azeotropic distillation. Where azeotropic distillation is desired, one or more distillation solvents suitable for the azeotropic distillation of a (meth)acrylic acid stream may be used in either or both of the distillation columns **10, 12.** In one embodiment, for example, the solvent is substantially water insoluble, generally having a solubility, in water at room temperature, of 0.5 weight percent or less, preferably 0.2 weight percent or less. Suitable examples of such a water insoluble solvent include, but are not limited to heptane; heptene; cycloheptane; cycloheptene; cycloheptatriene; methylcyclohexane; ethylcyclopentane; 1,2-dimethylcyclohexane; ethylcyclohexane; toluene; ethylbenzene; ortho-, meta-, or para- xylene; trichloroethylene; trichloropropene; 2,3-dichlorobutane; **1**-chloropentane; 1-chlorohexane; and **1-**chlorobenzene. In another embodiment, the solvent is selected from ethyl acetate, butyl acetate, dibutyl ether, hexane, heptane, ethyl methacrylate, diethyl ketone, methyl propyl ketone, methyl isobutyl ketone, and methyl tert-butyl ketone. In a further embodiment, the distillation solvent is a mixed solvent which includes at least two solvents. Suitable examples of solvents useful in such mixed solvent include, but is not limited to, diethyl ketone, methyl propyl ketone, methyl isobutyl ketone, methyl tert-butyl ketone, isopropyl acetate, n-propyl acetate, toluene, heptane and methylcyclohexane. The preferred distillation solvent is toluene.

Emanating from the bottom of the product column **10** is a (meth) acrylic acid stream **11** which is substantially free of water. Generally, the (meth)acrylic acid stream **11** has less than 1000, preferably less than 800, more preferably less than 500 ppm of water. The acrylic acid stream may also contain insubstantial amounts of at least one of the following: acetic acid, propionic acid, β-acryloxypropionic acid (AOPA), acrolein, furfural, benzaldehyde, maleic acid, maleic anhydride, protoanemonin, and acetaldehyde.

The (meth)acrylic acid stream **11** is suitable for use as a raw material in (meth)acrylic ester or (meth)acrylate polymer production. The (meth)acrylic acid may be used as is or be further processed, induing but not limited, additional distillation to remove specific impurities and further processing to form various grades of (meth)acrylic acid.

As shown in Figure 1, the intermediate waste stream **13** may then be fed to the acetic acid recovery column **12** to recover acetic acid before disposal or recycle of the stream **13** as wastewater. More particularly, the intermediate waste stream **13** may be distilled in the acetic acid recovery column **12** to produce a second wastewater stream **14** comprising more water, acetic acid and other organic compounds, and a bottoms stream **15** comprising (meth)acrylic acid. The bottoms stream **15** maybe recycled back to the product column **10** to recapture (meth)acrylic acid product, as shown in Figure 1.

The second wastewater stream **14** may then be recycled and used elsewhere in the process, or discarded and sent to a water treatment facility. However, it would be advantageous to strip at least some of the organic compounds, such as acetic acid, out of the second wastewater stream **14** prior to recycling to prevent accumulation of the organic compounds in the process. Where the second wastewater stream **14** is sent for treatment and ultimate disposal, removing some of the organic compounds first will lessen the cost of treatment, and thereby, decrease the cost of disposing of this process waste stream.

Thus, in accordance with the method of the present invention, as shown in Figure 1, at least a portion of the organic compounds in the second wastewater stream **14** are removed in a stripping column **16** by contacting the second wastewater stream **14** with a stripping gas **7** comprising at least a portion of the absorber off-gas stream **8.** This stripping of the wastewater stream **14,** using at least a portion of the absorber off-gas stream **8** as the stripping gas, produces a stripped wastewater stream **17** containing less organic compounds than the preceding overhead wastewater stream **14,** which is less costly to treat and discard. Also produced is an enriched waste gas stream **18** which contains more acetic acid and other organic compounds than the preceding absorber off-gas **8.** The enriched waste gas stream **18** from the stripping column **16** is generally sent to waste treatment for incineration and/or oxidation and then released to the atmosphere. Where only a portion of the absorber off-gas stream **8** is used as the stripping gas in the stripping column **16,** all or part of the other portion 23 of the absorber off-gas stream **8** may be recycled, such as to an oxidation reactor (not shown).

The stripping column **16** may be any column suitable for stripping undesirable organic components from wastewater. Such columns are known in the art and include packed columns and tray-containing columns.

Generally, the gas used as the stripping gas **7** in the stripping column **16** should have a water content of from 0 to 100 %, preferably 5 to 30 %, more preferably 8 to 20 % by weight, and a temperature from 20 °C to 250 °C, preferably 45 °C to 125 °C, more preferably from 50 °C to 90 °C. The use of at least a portion of the absorber off-gas stream **8** as a stripping gas **7** is advantageous because the absorber off gas emerges from the absorber with a sufficient heat and water content for adequately stripping undesirable components from the wastewater. Accordingly, treatment of a potential waste gas stripping stream, i.e., heating and adding or removing water, is avoided.

However, at times it may be desirable to either use other stripping gas streams such as fresh air, combustion air, other waste gas streams, etc., either alone or in combination with all or a portion of an absorber off-gas stream **8.** This may require additional heating of the stripping gas **7** to obtain a proper operating temperature range. Also, additional heat may be required to increase the removal of organics from the wastewater stream **14.** Consequently, a live steam sparge, external or internal reboilers, stripping gas feed pre-heater, or other methods known in the art of supplying additional heat to a stripping column may be utilized. In addition, there may be instances wherein additional momentum transfer within the stripping column is required. For example, where there is an increased pressure drop within the column and absorber pressure is insufficient to provide adequate momentum transfer. Consequently, devices such as a blower may be utilized to increase such momentum transfer.

One or more polymerization inhibitors may be introduced into the production process at various points such as polymerization inhibitor feeds **20, 21** and **22** shown in Figure 1. The polymerization inhibitor may include a water soluble or alcohol soluble polymerization inhibitor. Suitable examples include but are not limited to, hydroquinone; 4-methoxy phenol; 4-ethoxyphenol; 1,2-dihydroxybenzene; catechol monobutyl ether; pyrogallol; 4-aminophenol; 2-mercaptophenol; 4-mercaptophenol; 4-hydroxy-2,2,6,6-tetramethylpiperidinyloxy, free radical; 4-oxo-2,2,6,6-tetramethylpiperidinyloxy, free radical; 4-amino-2,2,6,6-tetramethylpiperidinyloxy, free radical; isomers thereof; derivatives thereof; mixtures of two or more thereof; or mixtures of one or more of the above with molecular oxygen.

With reference back to Figure 1, all or a portion of the wastewater stream **4** may be derived from the wastewater streams **14, 17** and fed with the aqueous stream **3** to the fractional absorber **2.** For example, the distillation overhead wastewater stream **14** may be stripped of undesirable components in a stripping column **16** using a stripping gas stream **7,** which includes absorber off-gas **8,** to produce a stripped wastewater stream **17.** All or a portion (stream **19,** shown in phantom) of the wastewater stream **17,** may then combined with the aqueous stream **3** and fed to the fractional absorber **2.** The amount of wastewater stream **4** which is stripped or unstripped will vary according to the amount of undesirable components in the available wastewater streams.

Efficacy of the method of the present invention is demonstrated by the following examples.

### EXAMPLES

Gas stripping experiments were performed in the laboratory using a water saturated N₂ stream to mimic the absorber off-gas (AOG) stream. This water-gas stream was preheated prior to introducing it into a stripping column at temperatures typical of a commercial-scale AOG stream. The experiments showed that organic compounds in the feed stream which simulated a wastewater process stream, were removed and absorbed into the water-gas (AOG) stream, and that the stripped bottoms stream from the stripper had a lower concentration of organic compounds. Details of the laboratory experiments are as follows.

A simulated wastewater feed containing acrylic acid (4.875 wt.%), acetic acid (4.657 wt.%) and water (91 wt.%) was prepared in a large container. The container was placed on a balance and a pump system was attached to it. N₂ was metered in and preheated in a bath set at 70 °C and fed to the bottom of a shell and tube heat exchanger packed with stainless steel Raschig rings. Water (25 mol% based on N₂ flow) was pumped into the exchanger system and the resulting flow was fed to the column system.

The column system was a 10-tray 1" Oldershaw system with a 100 mL bottoms flask. The feed was added at the top tray at a rate of 600 grams per hour (g/h). The system was operated at constant liquid level in the bottoms. Hydroquinone (HQ) is used to inhibit polymerization in the simulated wastewater feed.

The N₂ flow rates were metered in at 8 liters per minute (L/min), 13 L/min, 20 L/min, 28 L/min. The overheads and bottoms were collected hourly and analyzed for organic and water content.

In a typical experiment, the exchangers are preheated under N₂ and once the exit temperature reaches 60 °C, water is added to the exchanger and the heater is controlled by the sump target temperature (65 °C). The column is allowed to gain heat and once the top section (Tray 8) reaches 45 °C the feed is then introduced. The bottoms are removed to maintain a constant level and target flow. The cuts from the first hour are typically discarded and the bottoms and overheads are then collected hourly. An example of the data from one experimental run as described above are provided in the following Tables

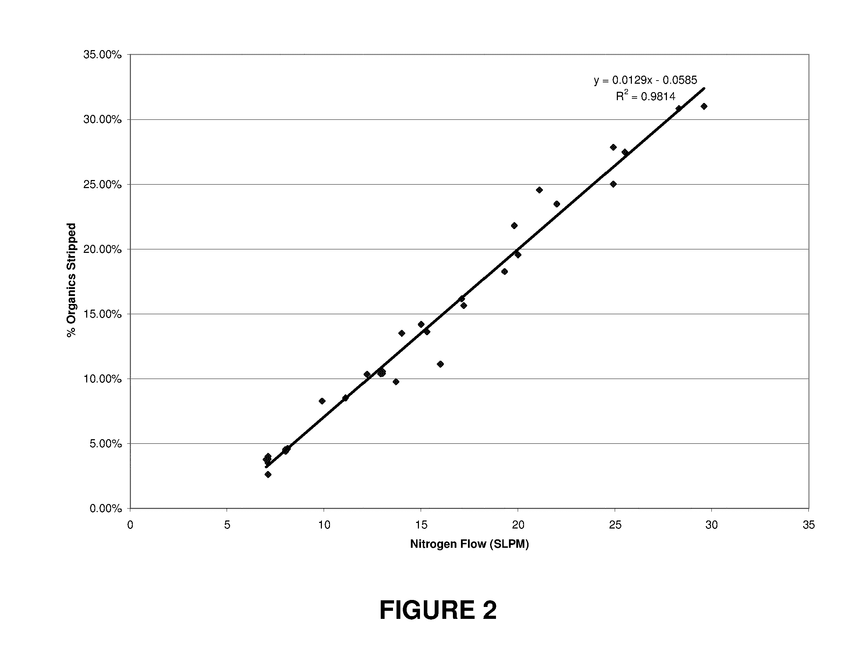

Under the conditions of 8 and 13 L/min N2 flow, the resulting stripping efficiency was 4.6% (combined AA and AcOH) and at 13 L/min the stripping efficiency was10.4% (combined). As the flow was increased, the stripping efficiency showed a near linear increase (see chart below) with a 30% value at 28 L/min flow, as shown in Figure 2. The stripping efficiency is defined as a ratio (%) of [organics overhead]/[organics fed]).

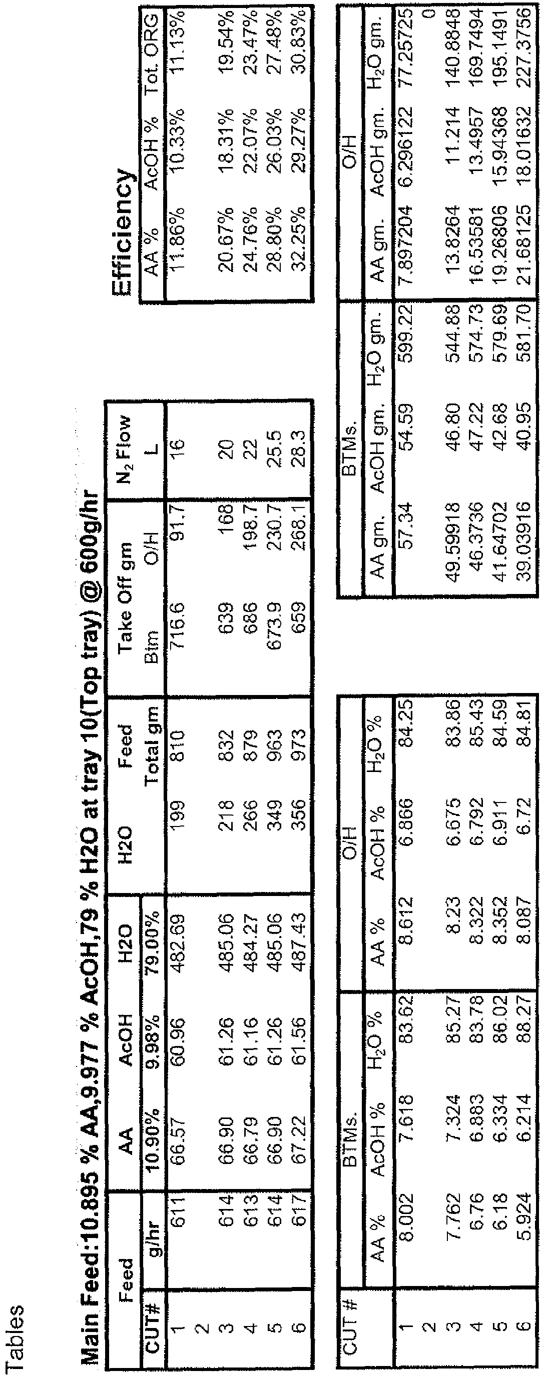

## Claims

1. A method for removing acetic acid from a waste water stream which is produced during purification of (meth)acrylic acid, said method comprising:
A) performing fractional absorption with a mixed gas comprising (meth)acrylic acid, acetic acid, propylene and acrolein to produce an aqueous product stream comprising (meth)acrylic acid, water and acetic acid, and an absorber off-gas stream comprising propylene and acrolein;
B) distilling the aqueous product stream to produce a purified (meth)acrylic acid stream and a waste water stream comprising water and acetic acid; and
C) contacting the absorber off gas and the waste water stream whereby at least a portion of the acetic acid moves from the waste water stream to the absorber off gas to produce a stripped waste water stream having a decreased acetic acid content and an enriched absorber off gas having an increased acetic acid content.

2. The method according to Claim 1, wherein the stripped waste water stream is discarded.

3. The method according to Claim 1, wherein the stripped waste water stream is subjected to further processing to remove or render inert one or more components therein.

4. The method according to Claim 1, wherein at least a portion of the stripped waste water stream is fed to at least one other process step.

5. The method according to Claim 1, wherein the enriched absorber off gas stream is provided to a thermal oxidizer.

6. The method according to Claim 1, wherein said mixed gas is derived from a vapor phase catalytic oxidation reaction and comprises 25 to 29 weight % (meth)acrylic acid and 1 to 3 weight % acetic acid.

7. The method according to Claim 1, wherein the aqueous product stream comprises from 70 to 93 weight % (meth)acrylic acid.

8. The method according to Claim 1, wherein the distilling step is accomplished in at least two distillation columns.

9. The method according to Claim 1, wherein the waste water stream comprises from 60 to 90 weight % water.
